# EUROPÄISCHE PATENTANMELDUNG

(11) **EP 4 173 557 A1**
(43) Veröffentlichungstag der Anmeldung: **03.05.2023**
(21) Anmeldenummer: 22202101.6
(22) Anmeldetag: 18.10.2022
(51) Int. Cl.: A61B 5/024, A61B 5/0205, G16H 40/63, A61B 5/00

(54) **VERFAHREN ZUM ÜBERPRÜFEN EINER MENSCHLICHEN KÖRPERFUNKTION**

(30) Priorität: 02.11.2021 DE 102021212331
(71) Anmelder: Sivantos Pte. Ltd., Singapore 539775 (SG)
(72) Erfinder: KRIEG, Julius, 91058 Erlangen (DE)
(74) Vertreter: FDST Patentanwälte

(57) **Zusammenfassung**

Die Erfindung nennt ein Verfahren zum Überprüfen einer menschlichen Körperfunktion, wobei mittels eines ersten Sensors (21) eines am Kopf (3) tragbaren Systems (2) an einer lateralen ersten Seite (9) eines Kopfes (3) eine Messung einer ersten Kenngröße (23) durchgeführt wird, welche Aufschluss über einen an besagter ersten Seite (9) des Kopfes (3) abgegebenen Schweiß gibt, wobei mittels eines zweiten Sensors (22) des besagten Systems (2) an einer der ersten Seite (9) gegenüberliegenden zweiten Seite (10) des Kopfes (3) eine Messung einer zweiten Kenngröße (24) durchgeführt wird, welche Aufschluss über einen an besagter zweiter Seite (10) des Kopfes (3) abgegebenen Schweiß gibt, und wobei anhand der ersten Kenngröße (23) und der zweiten Kenngröße (24) eine finale Kenngröße (50) für eine unilaterale Anhidrose eines Trägers des besagten Systems (6) ermittelt wird.

## Beschreibung

Die Erfindung betrifft ein Verfahren zum Überprüfen einer menschlichen Körperfunktion, wobei mittels eines ersten Sensors eines am Körper befestigbaren Systems an einer ersten Stelle eines Körpers eine Messung einer ersten Kenngröße durchgeführt wird, wobei mittels eines zweiten Sensors des besagten Systems an einer zweiten Stelle des Körpers eine Messung einer zweiten Kenngröße durchgeführt wird, und wobei anhand der ersten Kenngröße und der zweiten Kenngröße eine finale Kenngröße für eine Körperfunktion wird.

Das sog. Horner-Syndrom ist eine besondere Schädigung von Nerven des sympathischen Grenzstrangs im Bereich des Kopfes und des Halses. Die Schädigungen, welche dabei zum Horner-Syndrom führen, betreffen meist nur eine Seite der Nervenbahnen und Ganglien. Die Symptome des Horner-Syndroms beinhalten infolge einer Beeinträchtigung des für ein Auge zuständigen oberen Halsganglions meist eine Pupillenverengung (Miosis), das Herabhängen des Oberlids (Ptosis) und eine (scheinbare) Retraktion des Augapfels in die Augenhöhle (Pseudo-Enophthalmus), und treten dabei meist einseitig auf. Überdies sind oftmals die Bewegung des Augenlids bzw. der Pupille generell gehemmt (etwa beim ausbleibenden ziliospinalen Reflex, also der ausbleibenden ipsilateralen Erweiterung der Pupille nach einem stärkeren Schmerzreiz im Gesichts-, Schulter- oder Nackenbereich).

Eine Erkennung des Horner-Syndroms ist insbesondere deshalb wichtig, da dieses oft ein Symptom einer anderen zugrunde liegenden Erkrankung darstellt. Zu diesen gehören eine Durchtrennung einer Halsschlagader (Arteria carotis communis), einer inneren Schlagader (Arteria carotis interna) oder einer Wirbelarterie (Arteria vertebralis), das Wallenberg-Syndrom (ein Infarkt, bei welchem eine Wirbelarterie oder eine Arteria cerebelli inferior posterior blockiert ist), oder auch Verletzungen im Bereich der Hals- oder Brustwirbelsäule. Zudem können Tumorerkrankungen wie Schilddrüsenkrebs, Neuroblastom oder ein sog. Pancoast-Tumor ein Horner-Syndrom hervorrufen.

Während die drei erstgenannten, meist lebensgefährlichen Verletzungen eine unmittelbare medizinische Versorgung erfordern, liegt bei den Tumoren die Gefahr eher darin, diese nicht rechtzeitig zu erkennen. Dies gilt insbesondere für den Pancoast-Tumor, ein sich rasch ausbreitendes und dann auch auf andere Organe übergreifendes Bronchialkarzinom, welches meist jedoch ohne lungentypische Symptome wie schwerer Husten o.ä. verläuft und daher im Frühstadium - vor einem Übergreifen auf andere Organe - besonders schwer zu detektieren ist. Somit ist gerade hier eine zuverlässige Erkennung eines Horner-Syndroms von hoher Wichtigkeit.

Neben den o.g., meist einseitig auftretenden Beeinträchtigungen der Augen beim Horner-Syndrom tritt häufig noch ein einseitiges Abschwächen bzw. Ausbleiben der Schweißsekretion, eine sog. unilaterale Anhidrose auf. Eine übliche Untersuchung auf ein Horner-Syndrom beinhaltet meist die vier Kriterien Miosis, Ptosis, (Pseudo-)Enophthalmus (s.o.) sowie besagte unilaterale Anhidrose. Von den genannten Kriterien ist die unilaterale Anhidrose für einen behandelnden Arzt meist am schwierigsten festzustellen.

Der Erfindung liegt daher die Aufgabe zugrunde, ein Verfahren anzugeben, welches dazu geeignet ist, eine Erkennung einer unilateralen Anhidrose zu unterstützen. Der Erfindung liegt weiter die Aufgabe zugrunde, ein Gerät anzugeben, mittels dessen sich eine unilaterale Anhidrose besonders einfach erkennen lässt.

Die erstgenannte Aufgabe wird erfindungsgemäß gelöst durch ein Verfahren zum Überprüfen einer menschlichen Körperfunktion, wobei mittels eines ersten Sensors eines am Kopf tragbaren Systems an einer lateralen ersten Seite eines Kopfes eine Messung einer ersten Kenngröße durchgeführt wird, welche Aufschluss über einen an besagter ersten Seite des Kopfes abgegebenen Schweiß gibt, wobei mittels eines zweiten Sensors des besagten Systems an einer der ersten Seite gegenüberliegenden zweiten Seite des Kopfes eine Messung einer zweiten Kenngröße durchgeführt wird, welche Aufschluss über einen an besagter zweiter Seite des Kopfes abgegebenen Schweiß gibt, und wobei anhand der ersten Kenngröße und der zweiten Kenngröße eine finale Kenngröße für eine unilaterale Anhidrose eines Trägers des besagten Systems ermittelt wird. Vorteilhafte und teils für sich gesehen erfinderische Ausgestaltungen sind Gegenstand der Unteransprüche und der nachfolgenden Beschreibung.

Die zweitgenannte Aufgabe wird erfindungsgemäß gelöst durch eine Anordnung zum Überprüfen einer menschlichen Körperfunktion, umfassend ein am Kopf tragbares System mit einem ersten Sensor und einem zweiten Sensor und eine Steuereinheit, wobei der erste Sensor im bestimmungsgemäßen Betrieb des Systems auf einer lateralen ersten Seite eines Kopfes eines Trägers angeordnet ist, und der zweite Sensor auf einer der ersten Seite gegenüberliegenden zweiten Seite, wobei der erste Sensor und der zweite Sensor dazu eingerichtet sind, jeweils eine erste Kenngröße bzw. zweite Kenngröße zu messen, welche Aufschluss über einen an besagter ersten Seite bzw. zweiten Seite des Kopfes abgegebenen Schweiß gibt, und wobei die Steuereinheit dazu eingerichtet ist, anhand der ersten Kenngröße und der zweiten Kenngröße eine finale Kenngröße für eine unilaterale Anhidrose eines Trägers des besagten Systems zu ermitteln.

Die erfindungsgemäße Anordnung teilt die Vorzüge des erfindungsgemäßen Verfahrens. Die für das Verfahren und für seine Weiterbildungen angegebenen Vorteile können sinngemäß auf die Anordnung übertragen werden.

Als ein am Kopf tragbares System ist hierbei insbesondere ein binaurales Hörsystem mit einem ersten und einem zweiten Hörinstrument umfasst, welche vom Träger jeweils an einem und am anderen Ohr zu tragen sind, wobei der erste Sensor im ersten Hörinstrument und der zweite Sensor im zweiten Hörinstrument angeordnet ist. Ein derartiges binaurales Hörsystem mit zwei Hörgeräten weist meist von sich aus bereits den ersten und den zweiten Sensor für das Messen der beiden Kenngrößen auf.

Die Hörinstrumente können dabei insbesondere gegeben sein durch Hörgeräte im engeren Sinn, welche zur Versorgung einer Hörschwäche des Trägers vorgesehen und eingerichtet sind.

Die Hörinstrumente können aber auch durch Kopfhörer gegeben sein, welche primär für die Wiedergabe von Audioinhalten (wie z.B. Musik- oder Sprachdateien), aber auch zur Telekommunikation (mittels entsprechender Mikrofone in den Hörinstrumenten) vorgesehen sind. Als ein am Kopf tragbares System kann jedoch insbesondere auch eine mit den entsprechenden Sensoren ausgerüstete Brille, bevorzugt eine sog. Datenbrille (wie z.B. "Google Glass") gegeben sein.

Der erste Sensor ist dabei bevorzugt so angeordnet, dass er auf seiner betreffenden Seite des Kopfes eine Messung im Bereich des Ohres oder hinter dem Ohr durchführt. Für den zweiten Sensor gilt auf der anderen Kopfseite Entsprechendes.

Unter einer Messung einer Kenngröße, welche Aufschluss über einen an der entsprechenden Seite des Kopfes abgegebenen Schweiß gibt, ist insbesondere zu verstehen, dass eine durch den betreffenden Sensor eine physikalische Größe ermittelt wird, welche in einem quantitativen Zusammenhang mit der Schweißsekretion der Haut im Bereich des Sensors steht, also bzw. ein elektrischer Widerstand oder eine Impedanz der Haut (d.h., eine elektrodermale Aktivität), eine Hautreflektanz, oder auch eine Feuchtigkeit im Ohr. Die Kenngröße kann dann entweder durch die physikalische Größe selbst oder eine hieraus abgeleitete Größe für die Schweißsekretion, welche ggf. entsprechend für den Zweck skaliert wird, gegeben sein.

Die finale Kenngröße kann dabei einen kontinuierlichen Wertebereich aufweisen, z.B., als ein Wahrscheinlichkeitswert, oder als eine ggf. skalierte Differenz oder Korrelation der ersten und der zweiten Kenngröße (oder als eine stetig monotone Funktion der besagten Differenz bzw. Korrelation). Mit steigender Differenz bzw. abnehmender Korrelation steigt somit die Wahrscheinlichkeit für eine unilaterale Anhidrose.

Die finale Kenngröße kann jedoch auch als ein binärer Wert gegeben sein, indem z.B. für die Bildung der finalen Kenngröße eine Differenz oder Korrelation der erste und der zweiten Kenngröße mit einem entsprechenden Grenzwert verglichen werden, und bei einem Überschreiten eines geeignet zu wählenden oberen Grenzwertes für die Differenz bzw. bei einem Unterschreiten eines unteren Grenzwertes für die Korrelation auf ein hinreichend sicheres Vorliegen einer unilateralen Anhidrose geschlossen werden. Dies kann beispielsweise anhand eines Quotienten aus der ersten und der zweiten Kenngröße zu einem gegebenen Zeitpunkt erfolgen.

Elektronische Geräte, welche von einem Träger direkt am Körper getragen werden, weisen zunehmend komplexere Sensoren auf, mittels derer beim Betrieb eines solchen Geräts die zunehmende Überwachung bestimmter Körperfunktionen ermöglicht wird. Insbesondere am Kopf zu tragende Geräte, wie z.B. Hörinstrumente und dabei insbesondere Hörgeräte, welche zur Versorgung einer Hörschwäche des Trägers vorgesehen und eingerichtet sind, aber auch Datenbrillen oder spezielle Headset-Stirnbänder weisen zunehmend mehr Sensoren auf, mittels derer sich einzelne Körperfunktionen überwachen lassen.

Mittels eines derartigen, am Kopf tragbaren Systems, welches also zwei Hörinstrumente oder eine Datenbrille bzw. ein Headset-Stirnband umfassen kann, ist bei entsprechender Ausstattung des Systems mit dem besagten ersten und zweiten Sensor an geeigneten Stellen eine insbesondere wiederholte oder auch laufende Messung der besagten physikalischen Kenngrößen möglich, welche einen Rückschluss auf die Schweißsekretion auf der linken und der rechten Kopf- und/oder Halsseite erlauben, und somit der ersten und der zweiten Kenngröße zugrunde liegen.

Oftmals weisen die genannten Geräte geeignete Sensoren für besagte Messung bereits für andere Zwecke auf, sodass nicht einmal eine zusätzliche Umgestaltung der Konstruktion für die Durchführung des Verfahrens erforderlich ist.

Das Verfahren ermöglicht es nun, eine bereits vorhandene Infrastruktur bei den genannten Gerätetypen für die insbesondere wiederholte oder laufende Messung der ersten und der zweiten Kenngröße zu nutzen, sodass eine sehr zuverlässige Aussage darüber getroffen werden kann, ob eine unilaterale Anhidrose vorliegt. Alternativ oder auch zusätzlich dazu kann einem Mediziner eine sehr aussagekräftige finale Kenngröße - z.B. in Form eines Wahrscheinlichkeitswertes - für eine unilaterale Anhidrose zur Verfügung gestellt werden.

Eine derartige Aussage bzw. aussagekräftige finale Kenngröße kann zudem ohne das Zutun eines Arztes oder einer Pflegeperson erstellt werden, sodass die Aussage bzw. finale Kenngröße bei einer Untersuchung durch einen Arzt bzw. durch eine Pflegeperson bereits vorliegen kann.

Bevorzugt erfolgt die Messung der ersten bzw. der zweiten Kenngröße jeweils als eine Messung der elektrodermalen Aktivität, also insbesondere einer elektrischer Widerstand oder Impedanz der Haut, und/oder als eine Messung der Hautreflektanz auf der ersten bzw. zweiten Seite des Kopfes. Durch eine Schweißsekretion werden an der betreffenden Stelle eine elektrische Leitfähigkeit und eine Reflektanz (ein Reflexionsgrad für einfallendes Licht) erhöht.

Für den Fall, dass ein binaurales Hörsystem mit einem ersten und einem zweiten Hörinstrument verwendet wird, ist es vorteilhaft, wenn für das erste Hörinstrument und/oder das zweite Hörinstrument jeweils ein Grad einer Passfestigkeit ermittelt wird, wobei die erste Kenngröße bzw. die zweite Kenngröße zusätzlich anhand des zugehörigen Grades der Passfestigkeit ermittelt wird, und wobei der Grad der Passfestigkeit für jedes der beiden Hörinstrumente jeweils ermittelt wird anhand eines kapazitiven Sensors und/oder eines photoplethysmographischen Sensors und/oder eines Drucksensors und/oder wenigstens einer Transferfunktion, welche eine Propagation eines Schallsignals von einem Lautsprecher des jeweiligen Hörinstrumentes zu einem Mikrofon desselben Hörinstrumentes repräsentiert, oder welche einen Unterschied zwischen einem Mikrofon des jeweiligen Hörinstrumentes und einem weiteren Mikrofon desselben Hörinstrumentes repräsentiert (sog. relative Transferfunktion eines Mikrofons zum anderen Mikrofon), und/oder zweier Transferfunktionen, welche eine Propagation eines Schallsignals von einem Lautsprecher des jeweiligen Hörinstrumentes zu zwei unterschiedlichen Mikrofonen desselben Hörinstrumentes repräsentieren. Bevorzugt sind im letztgenannten Fall zweier Mikrofone (und einer relativen Transferfunktion der beiden Mikrofone oder der beiden jeweiligen Transferfunktionen für die Schallpropagation von einem Lautsprecher aus) diese voneinander räumlich getrennt.

Wird dabei für eines der beiden Hörinstrumente ein reduzierter Grad der Passfestigkeit ermittelt, sodass also das betreffende Hörinstrument nicht optimal an seiner bestimmungsgemäßen Tragestelle am Gehörgang einsitzt, so ist auch der Kontakt des zugehörigen Sensors mit den angrenzenden Hautpartien reduziert. Die betreffende erste oder zweite Kenngröße kann dann entsprechend des ermittelten Grades der Passfestigkeit entsprechend skaliert werden. Der Grad der Passfestigkeit ist vorzugsweise auf physikalisch ähnliche Weise zu ermitteln ist wie die jeweilige Kenngröße (also z.B. eine kapazitive Messung der Passfestigkeit bei einer Messung der elektrodermalen Aktivität für die Kenngröße). Die Skalierung der jeweiligen Messgröße zum Ermitteln der ersten bzw. zweiten Kenngröße erfolgt dabei nicht notwendigerweise in linearer Abhängigkeit vom Grad der Passfestigkeit. Insbesondere kann der jeweilige Grad der Passfestigkeit auch dazu verwendet werden, einen vorab bestimmten, tabellierten Wert für die Skalierung zu ermitteln.

Im genannten Fall des binauralen Hörsystems erfolgt in einer vorteilhaften alternativen Ausgestaltung, oder auch zusätzlich zu o.g. Messverfahren, die Messung der ersten bzw. der zweiten Kenngröße jeweils als eine Messung einer Feuchtigkeit im betreffenden Gehörgang und/oder als eine Messung einer Cerumen-Kontamination des jeweiligen Hörinstruments. Hörinstrumente, und dabei insbesondere Hörgeräte im engeren Sinn, weisen oftmals einen Sensor zur Messung einer Feuchtigkeit im Gehörgang auf. Dies kann z.B. für ein aktives Venting-System der Fall sein. Eine Cerumen-Kontamination kann dabei auch in einem Ladegerät der Hörinstrumente ermittelt werden. In diesem Fall ist der jeweilige Sensor des am Kopf tragbaren Systems dem System lediglich zugeordnet, und nicht unmittelbar im System angeordnet, bzw., das Ladegerät mit den entsprechenden Sensoren ist als ein Teil des Systems anzusehen, wobei lediglich die Hörinstrumente des Systems am Kopf tragbar sind.

Günstigerweise werden die erste Kenngröße und die zweite Kenngröße vom besagten System an ein externes Gerät übertragen, wobei die finale Kenngröße für die unilaterale Anhidrose des Trägers des Systems mittels des externen Gerätes ermittelt wird. Hierdurch können Rechenoperationen zum Ermitteln der finalen Kenngröße, sowie ggf. das Speichern von Werten der beiden Kenngrößen über einen längeren Zeitraum, auf das externe Gerät ausgelagert werden. Das externe Gerät kann dabei insbesondere gegeben sein durch ein Smartphone, eine Smartwatch, ein Fitnessarmband oder ein vergleichbares elektronisches Gerät, welches ein Benutzer für den bestimmungsgemäßen Betrieb am Körper trägt oder mit sich führt. In einer Ausgestaltung der Anordnung ist dabei also die Steuereinheit in einem derartigen externen Gerät angeordnet, welches mit dem am Kopf tragbaren System datentechnisch (z.B. über Bluetooth o.ä.) verbindbar ist.

Die finale Kenngröße für die unilaterale Anhidrose wird bevorzugt anhand eines Korrelationsmaßes ermittelt, welches anhand eines Wertes der ersten Kenngröße und eines Wertes der zweiten Kenngröße bestimmt wird.

Insbesondere kann die Messung der ersten und der zweiten Kenngröße zu einer Mehrzahl an Zeitpunkten durchgeführt werden, und die finale Kenngröße für die unilaterale Anhidrose anhand der Werte der ersten und der zweiten Kenngröße zu den besagten Zeitpunkten ermittelt werden.

Dies erfolgt bevorzugt, indem für wenigstens einige der besagten Zeitpunkte ein Korrelationsmaß, z.B. ein Korrelationskoeffizient und/oder eine Stichprobenvarianz, der betreffenden Werte der ersten Kenngröße und der zweiten Kenngröße ermittelt wird, und die finale Kenngröße für die unilaterale Anhidrose anhand des für die besagten Zeitpunkte ermittelten Korrelationsmaßes ermittelt wird. Beispielsweise werden für die besagten Zeitpunkte jeweils auf der linken und der rechten Seite des Kopfes bzw. des Halses die Werte der physikalischen Größe gemessen, die der ersten und der zweiten Kenngröße zugrunde liegt. Diese Werte können nun für jeden Zeitpunkt einzeln miteinander verglichen werden, oder ein Korrelationsmaß, z.B. eine Stichprobenkovarianz, kann für die beiden Zeitreihen gebildet werden, welche sich jeweils aus den Werten ergeben, die vom ersten bzw. zweiten Sensor gemessen werden. Für das Korrelationsmaß kann dabei insbesondere auch eine relative Abweichung herangezogen werden.

In einer vorteilhaften Ausgestaltung wird dabei die finale Kenngröße für die unilaterale Anhidrose als ein Wahrscheinlichkeitswert aus dem für die besagten Zeitpunkte ermittelten Korrelationsmaß ermittelt, oder als ein binärer Wert abhängig von einem Unterschreiten eines unteren Grenzwertes durch das für die besagten Zeitpunkte ermittelte Korrelationsmaß. Das Unterschreiten des unteren Grenzwertes als Kriterium kann hierbei als ein Einzelereignis für einen einzigen der Zeitpunkte oder eine bestimmte Mindestanzahl der Zeitpunkte definiert sein (z.B. ein Unterschreiten für mindestens drei aufeinanderfolgende Zeitpunkte oder fünf Zeitpunkte innerhalb eines vorgegebenen Zeitraumes, z.B. einer Stunde oder eines Tages), oder für ein Zeitmittel über einen vorgegebenen Zeitraum (z.B. für eine Stunde oder einen Tag oder eine Woche) definiert sein.

Zweckmäßigerweise wird eine Benachrichtigung an den Träger und/oder an eine Pflegeperson des Trägers und/oder einen ärztlichen Dienst ausgegeben, wenn die finale Kenngröße für die unilaterale Anhidrose einen kritischen Wert einnimmt. Unter einem ärztlichen Dienst ist hierbei insbesondere ein niedergelassener Arzt und ein Krankenhaus o.ä. umfasst, jedoch kann auch ein automatisierter Dienst gegeben sein (z.B. durch einen Server eines öffentlichen Pflege- oder Gesundheitssystems), welcher die entsprechende Benachrichtigung an einen Arzt oder eine Pflegeperson weiterleitet. Die Benachrichtigung an den Träger kann dabei im Fall eines binauralen Hörsystems insbesondere durch eine automatische Sprachausgabe über Lautsprecher der Hörinstrumente erfolgen.

In einer Ausgestaltung der Anordnung sind dabei der erste Sensor und/oder der zweite Sensor jeweils gegeben ist durch einen PPG-Sensor und/oder einen kapazitiven Sensor und/oder einen Elektromyographie-Sensor (EMG) und/oder einen Elektrokardiographie-Sensor (EKG) und/oder einen Elektroenzephalographie-Sensor (EEG) und/oder einen Feuchtigkeitssensor und/oder einen Hautleitfähigkeitssensor (electrodermal activity, "EDA" bzw. galvanic skin response, "GSR" und/oder einen Reflektometriesensor und/oder einen Cerumen-Sensor und/oder eine Anordnung, welche eine Lichtquelle und einen Photodetektor aufweist. Ein Rückschluss auf eine Schweißsekretion kann dabei insbesondere über das Messen, Ermitteln und/oder Ableiten eines Hautwiderstandes und/oder einer reflektierten Lichtmenge aus den jeweiligen Messdaten erfolgen.

Nachfolgend wird ein Ausführungsbeispiel der Erfindung anhand von Zeichnungen näher erläutert. Hierbei zeigen jeweils schematisch:
- Fig. 1: in einem Blockschaltbild eine Anordnung aus einem binauralen Hörsystem mit zwei Hörgeräten und einem Smartphone, welche zum Überprüfen eines Trägers des Hörsystems auf eine unilaterale Anhidrose eingerichtet ist, und
- Fig. 2: in einem Blockdiagram ein Verfahren zum Überprüfen eines Trägers des Hörsystems nach Fig. 1 auf eine unilaterale Anhidrose hin.

Einander entsprechende Teile und Größen sind in allen Figuren jeweils mit denselben Bezugszeichen versehen.

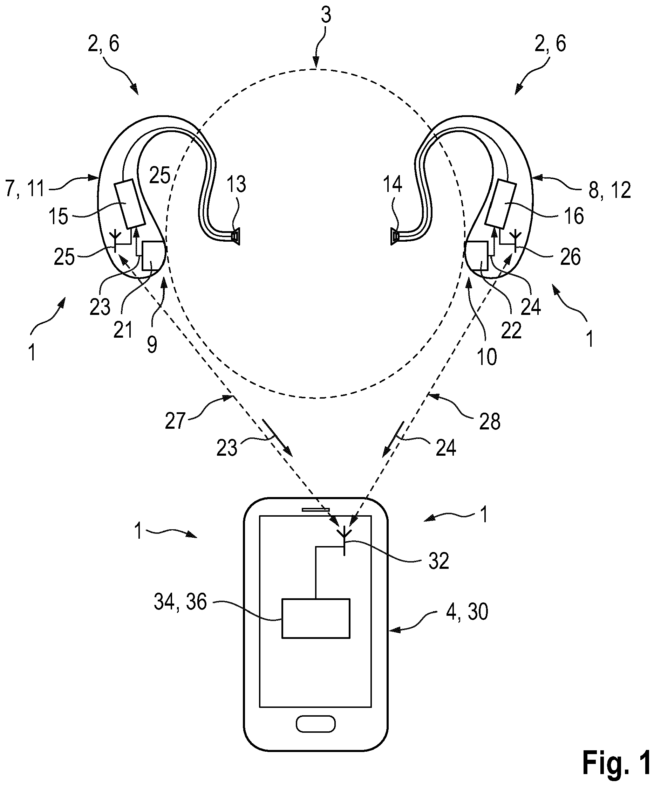

In Figur 1 ist schematisch in einem erweiterten Blockschaltbild eine Anordnung 1 dargestellt, welche eine durch eine nicht näher gezeigten Benutzer an seinem Kopf 3 zu tragendes (tragbares) System 2 und ein mit dem am Kopf 3 tragbaren System 2 datentechnisch verbindbares externes Gerät 4 umfasst. Der Kopf 3 (gestrichelt) ist hierbei in Fig. 1 lediglich angedeutet. Das am Kopf 3 tragbare System 2 ist vorliegend gegeben durch ein binaurales Hörsystem 6 mit einem ersten Hörinstrument 7 und einem zweiten Hörinstrument 8. Das erste und das zweite Hörinstrument 7, 8 sind vorliegend jeweils gegeben durch ein erstes Hörgerät 11 bzw. ein zweites Hörgerät 12, welche im bestimmungsgemäßen Betrieb des binauralen Hörsystems 6 vom besagten Benutzer, also dem Träger des binauralen Hörsystems 6, jeweils am linken bzw. rechten Ohr zu tragen sind (der Zuordnung nach kann dabei das erste Hörgerät 11 entweder am linken oder am rechten Ohr getragen werden). Das erste Hörinstrument 7 und das zweite Hörinstrument 8 könnten genauso gut auch durch Kopfhörer eines Kopfhörersystems bzw. eines Headsets gegeben sein.

Das erste Hörgerät 11 weist eine ersten Lautsprecher 13 auf, welcher mit einer ersten Prozessoreinheit 15 des ersten Hörgerätes 11 verbunden ist, und ein von dieser ausgegebenes Ausgangssignal in einen in entsprechenden Ausgangsschall umwandelt. Analog dazu weist das zweite Hörgerät 12 einen zweiten Lautsprecher 14 auf, welcher mit einer zweiten Prozessoreinheit 16 des zweiten Hörgerätes 12 verbunden ist, und ebenso ein von dieser ausgegebenes Ausgangssignal in einen entsprechenden Ausgangsschall umwandelt. Darüber hinaus weisen das erste Hörgerät 11 und das zweite Hörgerät 12 noch weitere, in Figur 1 lediglich angedeutete Komponenten auf, welche im jeweiligen Hörgerät 11, 12 zur Versorgung einer Hörschwäche des Trägers des binauralen Hörsystems 6 vorgesehen sind, sodass besagte Versorgung durch das erste und das zweite Hörgerät 11, 12 erfolgen kann (und diese entsprechend eingerichtet sind).

Das erste Hörgerät 11 weist eine ersten Sensor 21 auf, welcher mit der ersten Prozessoreinheit 15 verbunden ist, und welcher dazu eingerichtet ist, beim bestimmungsgemäßen Tragen am Ohr an einer lateralen ersten Seite 9 des Kopfes 3 im Bereich des betreffenden Ohres in noch darstellender Weise eine erste Kenngröße 23 zu messen, welche Aufschluss über eine Schweißsekretion des Trägers an der betreffenden Messstelle der ersten Seite 9 gibt. Dies kann beispielsweise erfolgen über eine optische Messung der Hautreflextanz oder über eine Messung der elektrodermalen Aktivität, also eine Messung der Hautleitfähigkeit (bzw. des elektrischen Widerstands der Haut).

Analog dazu weist das zweite Hörgerät 12 einen zweiten Sensor 22 auf, welcher mit der zweiten Prozessoreinheit 16 verbunden ist, und welcher dazu eingerichtet ist, bei einem bestimmungsgemäßen Tragen des zweiten Hörgerätes 12 an einer der ersten Seite 9 gegenüber liegenden zweiten Seite 10 des Kopfes 3 eine zweite Kenngröße 24 zu messen, welche Aufschluss gibt über eine Schweißsekretion des Trägers an der zweiten Messstelle der zweiten Seite 10 (also zum Beispiel bei einem BTE-Hörgerät ein Bereich hinter dem Ohr). Bevorzugt sind der erste Sensor 21 und der zweite Sensor 22 identisch ausgestaltet (und verwenden also in beiden Fällen dasselbe physikalische Messprinzip). Das am Kopf tragbare System 2 könnte in einer nicht dargestellten Alternative beispielsweise auch durch eine Datenbrille gegeben sein, welche mit dem ersten und dem zweiten Sensor 21, 22 ausgerüstet ist.

Für die Messung der ersten und der zweiten Kenngröße 23, 24 können dabei in einer alternativen Ausgestaltung der Erfindung auch noch weitere Sensoren als der erste und der zweite Sensor 21, 22 herangezogen werden.

Die erste Kenngröße 23, welche vom ersten Sensor 21 gemessen wurde, wird nun in der ersten Prozessoreinheit 15 in ein übertragbares Datenformat übersetzt, und über erste Verbindung 27 zum externen Gerät 4, welche mittels einer mit der ersten Prozessoreinheit 15 verbundenen ersten Antenne 25 des ersten Hörgerätes 11 hergestellt wird, an das externe Gerät 4 übertragen. In vergleichbarer Weise wird die zweite Kenngröße 24 über eine zweite Verbindung 28, welche mittels einer mit der zweiten Prozessoreinheit 16 verbundenen zweiten Antenne 26 des zweiten Hörgerätes 12 hergestellt wird, ebenso an das externe Gerät 4 übertragen.

Das externe Gerät 4 ist vorliegend gegeben durch ein Smartphone 30 mit einer Antenne 32 und einer als Prozessoreinheit 34 ausgebildeten Steuereinheit 36, könnte aber auch als eine Smartwatch oder ein vergleichbares Gerät gegeben sein, welches für den bestimmungsgemäßen Betrieb insbesondere von Benutzer mitgeführt oder am Körper getragen wird. Die Prozessoreinheit 34 weist dabei wenigstens einen Prozessorkern und einen durch diesen adressierbaren Arbeitsspeicher sowie ggf. noch einen nichtflüchtigen Speicher zur Hinterlegung eines Betriebsprogramms und/oder -systems auf.

Die erste und die zweite Antenne 25, 26 sowie die Antenne 32 des Smartphones 30 stehen dabei generisch für entsprechende Kommunikationsrichtungen, welche dazu geeignet und eingerichtet sind, die erste und die zweite Verbindung 27, 28 zwischen dem ersten bzw. zweiten Hörgerät 11, 12 und dem Smartphone 30 aufzubauen.

In der Steuereinheit 36 werden in noch zu beschreibender Weise die erste und die zweite Kenngröße 23, 24 bewertet, um hinsichtlich einer unilateralen Anhidrose des Trägers des binauralen Hörsystems 6 eine finale Kenngröße auszugeben. Das Ermitteln der finalen Kenngröße kann dabei in einer alternativen Ausgestaltung der Erfindung auch im binauralen Hörsystem 6 selbst, z.B. in der ersten und/oder zweiten Prozessoreinheit 15, 16 als eine alternative Ausgestaltung (nicht dargestellt) zur Steuereinheit (36), oder auch in einer mit entsprechender Rechenkapazität versehenen und dazu eingerichteten Ladestation für das erste und das zweite Hörgerät 11, 12 erfolgen.

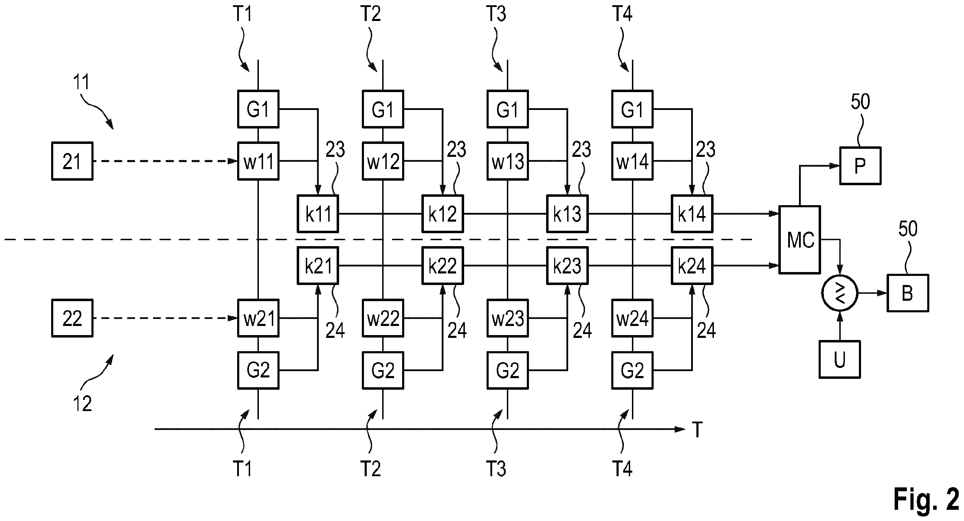

In Fig. 2 ist schematisch in einem Blockdiagramm gegen eine Zeitachse T ein Verlauf eines Verfahrens dargestellt, mittels dessen für den Träger des binauralen Hörsystems 6 nach Figur 1 eine finale Kenngröße für eine unilaterale Anhidrose feststellbar ist.

Zu einer Mehrzahl an Zeitpunkten T1, T2, T3, T4 werden dabei in der in Figur 2 dargestellten Ausgestaltung der Erfindung durch den ersten Sensor 21 und den zweiten Sensor 22 des ersten bzw. zweiten Hörgerätes 11, 12 jeweils Messwerte w11, w12, w13, w14 als eine Vorstufe für die erste Kenngröße 23 und Messwerte w21, w22, w23, w24 als eine Vorstufe für die zweite Kenngröße 24 gemessen. Die besagten Messwerte w11, ..., w24 können dabei z.B. durch konkrete Werte einer Hautreflektanz oder einer Leitfähigkeit der Haut gegeben sein (je nach physikalischer Ausgestaltung des ersten und des zweiten Sensors 11, 12).

Überdies wird für jedes der beiden Hörgeräte 11, 12 laufend ein Grad G1, G2 einer Passfestigkeit ermittelt. Dies kann bevorzugt jeweils zu den angegebenen Zeitpunkten T1, T2, T3, T4 erfolgen, also G1(T1), G2(T1), G1 (T2) etc. Der jeweilige Grad G1, G2 der Passfestigkeit kann z.B. jeweils ermittelt werden anhand eines kapazitiven Sensors (nicht dargestellt), welcher die Stärke eines Kontaktes des jeweiligen Hörgerätes 11, 12 mit der Haut im Bereich des zugehörigen ersten bzw. zweiten Sensors 21, 22 bestimmt, oder auch über einen PPG-Sensor, für welchen sich eine solche Stärke des Kontaktes anhand der Signalstärke abschätzen lässt.

Anhand des jeweiligen Grades G1 der Passfestigkeit des ersten Hörgerätes 11 werden nun die einzelnen Messwerte w11 - w14 entsprechend gewichtet, also mit einem entsprechenden Skalierungsfaktor multipliziert, welcher jeweils aus dem Grad G1 der Passfestigkeit ermittelt wird (z.B. anhand von vorab tabellierten Werten, welche durch Kalibrationsmessungen bestimmt werden), und so für die Mehrzahl an Zeitpunkten T1, T2, T3 und T4 die jeweiligen Werte k11, k12, k13 und k14 für die erste Kenngröße 23 ermittelt.

Auf vergleichbare Weise werden anhand des jeweiligen Grades G2 der Passfestigkeit des zweiten Hörgerätes 12 die einzelnen Messwerte w21 - w24 skaliert, und so die entsprechenden Werte k21, k22, k23 und k24 für die zweite Kenngröße 24 ermittelt.

In der in Figur 1 dargestellten Ausgestaltung der Erfindung werden die Werte k11, ..., k14 der ersten Kenngröße 23 und die Werte k21, ..., k24 für die zweite Kenngröße 24 direkt durch die vom ersten bzw. zweiten Sensor 21, 22 gemessenen Messwerte w11, ..., w14 bzw. w21, ..., w24 gebildet.

Aus den Werten k11, k12, k13 und k14 für die erste Kenngröße 23 zu den Zeitpunkten T1, T2, T3 und T4 sowie den entsprechenden Werten k21, k22, k23 und k24 für die zweite Kenngröße 24 wird nun ein Korrelationsmaß MC ermittelt. Das Korrelationsmaß kann dabei z.B. durch einen Korrelationskoeffizienten oder eine Stichprobenkovarianz der genannten Werte für die beiden Kenngrößen 23, 24 gebildet werden. Auch besteht eine Möglichkeit einer geeignet gewichteten Mittelung über mehrere Korrelationsgrößen, um das Korrelationsmaß MC zu erzeugen.

Sind dabei die Werte k11, ..., k14 für die erste Kenngröße 23 stark mit den Werten k21, ..., k24 für die zweite Kenngröße korreliert, so bedeutet dies, dass die Schweißsekretion auf beiden Seiten zu der Mehrzahl an Zeitpunkten T1, ..., T4 stark korreliert ist, und somit keine hohe Wahrscheinlichkeit für eine unilaterale Anhidrose vorliegt. Weisen jedoch die besagten Werte eine geringe Korrelation auf, so deutet dies darauf hin, dass die Schweißsekretion auf einer Seite unterdrückt oder gar erheblich unterdrückt ist, und somit unabhängig von einer körperlichen Anstrengung oder einer Ruhephase des Trägers verringert ist oder gar weitgehend unterbleibt.

Aus dem Korrelationsmaß MC wird nun daher ein Wahrscheinlichkeitswert P für ein Vorliegen einer unilateralen Anhidrose beim Träger des binauralen Hörsystems 6 ermittelt, indem das Korrelationsmaß MC in geeigneter Weise auf das Intervall [0, 1] abgebildet wird. Gilt auch für das Korrelationsmaß MC ∈ [0, 1], so kann z.B. eine antilineare Abbildung der Form P (MC) = 1 - MC verwendet werden; es kann hierbei jedoch eine monotone Skalierung durchgeführt werden, z.B. in der Form P (MC) = 1 - x² o.ä.

Der Wahrscheinlichkeitswert P kann nun als finale Kenngröße 50 für ein Vorliegen einer unilateralen Anhidrose verwendet werden. Alternativ dazu kann eine solche finale Kenngröße 50 für ein Vorliegen einer unilateralen Anhidrose auch als ein binärer Wert B aus dem Korrelationsmaß MC ermittelt werden. Der binäre Wert B nimmt den Wert 0 ein, wenn das Korrelationsmaß MC über einem unteren Grenzwert U liegt (und somit hinreichend hohe Korrelation der Werte k11, ..., k24 für die Kenngrößen 23, 24 auf beiden Seiten vorliegt), und den Wert 1 ein, wenn das Korrelationsmaß MC unter dem unteren Grenzwert U liegt, also U < MC (und somit die Schweißsekretion auf beiden Seiten des Kopfes unkorreliert ist, sodass von einem Ausfall auf einer Seite ausgegangen werden kann).

Obwohl die Erfindung im Detail durch das bevorzugte Ausführungsbeispiel näher illustriert und beschrieben wurde, so ist die Erfindung nicht durch die offenbarten Beispiele eingeschränkt; andere Variationen können vom Fachmann hieraus abgeleitet werden, ohne den Schutzumfang der Erfindung zu verlassen.

### Bezugszeichenliste

- 1: Anordnung
- 2: am Kopf tragbares System
- 4: externes Gerät
- 6: binaurales Hörsystem
- 7: erstes Hörinstrument
- 8: zweites Hörinstrument
- 9: erste Seite
- 10: zweite Seite
- 11: erstes Hörgerät
- 12: zweites Hörgerät
- 13: erster Lautsprecher
- 14: zweiter Lautsprecher
- 15: erste Prozessoreinheit
- 16: zweite Prozessoreinheit
- 21: erster Sensor
- 22: zweiter Sensor
- 23: erste Kenngröße
- 24: zweite Kenngröße
- 25: erste Antenne
- 26: zweite Antenne
- 27: erste Verbindung
- 28: zweite Verbindung
- 30: Smartphone
- 32: Antenne
- 34: Prozessoreinheit
- 36: Steuereinheit
- 50: finale Kenngröße

- B: binärer Wert
- G1: Grad einer Passfestigkeit des ersten Hörgerätes
- G2: Grad einer Passfestigkeit des zweiten Hörgerätes
- k11...k14: Wert (für die erste Kenngröße)
- k21...k24: Wert (für die zweite Kenngröße)
- MC: Korrelationsmaß
- P: Wahrscheinlichkeitswert
- T: Zeitachse
- T1...T4: Zeitpunkt
- U: Unterer Grenzwert
- w11 ...w14: Messwert (des ersten Sensors)
- w21 ...w24: Messwert (des zweiten Sensors)

## Patentansprüche

1. Verfahren zum Überprüfen einer menschlichen Körperfunktion,
wobei mittels eines ersten Sensors (21) eines am Kopf (3) tragbaren Systems (2) an einer lateralen ersten Seite (9) eines Kopfes (3) eine Messung einer ersten Kenngröße (23) durchgeführt wird, welche Aufschluss über einen an besagter ersten Seite (9) des Kopfes (3) abgegebenen Schweiß gibt,
wobei mittels eines zweiten Sensors (22) des besagten Systems (2) an einer der ersten Seite (9) gegenüberliegenden zweiten Seite (10) des Kopfes (3) eine Messung einer zweiten Kenngröße (24) durchgeführt wird, welche Aufschluss über einen an besagter zweiter Seite (10) des Kopfes (3) abgegebenen Schweiß gibt, und
wobei anhand der ersten Kenngröße (23) und der zweiten Kenngröße (24) eine finale Kenngröße (50) für eine unilaterale Anhidrose eines Trägers des besagten Systems (6) ermittelt wird.

2. Verfahren nach Anspruch 1,
wobei die Messung der ersten bzw. der zweiten Kenngröße (23, 24) jeweils als eine Messung der elektrodermalen Aktivität und/oder als eine Messung der Hautreflektanz auf der ersten bzw. zweiten Seite des Kopfes (9, 10) erfolgt.

3. Verfahren nach Anspruch 1 oder nach Anspruch 2,
wobei als das am Kopf (3) tragbare System (2) ein binaurales Hörsystem (6) mit einem ersten Hörinstrument (7) und einem zweiten Hörinstrument (8) verwendet wird,
wobei der erste Sensor (21) im ersten Hörinstrument (7) und der zweite Sensor (22) im zweiten Hörinstrument (8) angeordnet ist.

4. Verfahren nach Anspruch 3,
wobei für das erste Hörinstrument (7) und/oder das zweite Hörinstrument (8) jeweils ein Grad (G1, G2) einer Passfestigkeit ermittelt wird, wobei die erste Kenngröße (23) bzw. die zweite Kenngröße (24) zusätzlich anhand des zugehörigen Grades (G1, G2) der Passfestigkeit ermittelt wird, und
wobei der Grad (G1, G2) der Passfestigkeit für jedes der beiden Hörinstrumente (7, 8) jeweils ermittelt wird anhand
- eines kapazitiven Sensors und/oder
- eines photoplethysmographischen Sensors und/oder
- eines Drucksensors und/oder
- wenigstens einer Transferfunktion, welche eine Propagation eines Schallsignals von einem Lautsprecher (13, 14) des jeweiligen Hörinstrumentes (7, 8) zu einem Mikrofon desselben Hörinstrumentes (7, 8) repräsentiert, oder welche einen Unterschied zwischen einem Mikrofon des jeweiligen Hörinstrumentes (7, 8) und einem weiteren Mikrofon desselben Hörinstrumentes (7, 8) repräsentiert.

5. Verfahren nach Anspruch 3 oder Anspruch 4,
wobei die Messung der ersten bzw. der zweiten Kenngröße (23, 24) jeweils als eine Messung einer Feuchtigkeit im betreffenden Gehörgang und/oder als eine Messung einer Cerumen-Kontamination des jeweiligen Hörinstruments (7, 8) erfolgt.

6. Verfahren nach Anspruch 1 oder nach Anspruch 2,
wobei als das am Kopf (3) tragbare System (2) eine Brille, insbesondere Datenbrille, verwendet wird, und
wobei der erste Sensor (21) und der zweite Sensor (22) auf der linken bzw. rechten Seite der Brille angeordnet ist.

7. Verfahren nach einem der vorhergehenden Ansprüche,
wobei die erste Kenngröße (23) und die zweite Kenngröße (24) vom besagten System (2) an ein externes Gerät (4) übertragen werden, und
wobei die finale Kenngröße (50) für die unilaterale Anhidrose des Trägers des Systems (2) mittels des externen Gerätes (4) ermittelt wird.

8. Verfahren nach einem der vorhergehenden Ansprüche,
wobei die finale Kenngröße (50) für die unilaterale Anhidrose anhand eines Korrelationsmaßes (MC) ermittelt wird, welches anhand eines Wertes (k11-k14) der ersten Kenngröße (23) und eines Wertes (k21-k24) der zweiten Kenngröße (24) bestimmt wird.

9. Verfahren nach einem der vorhergehenden Ansprüche,
wobei die Messung der ersten und der zweiten Kenngröße (23, 24) zu einer Mehrzahl an Zeitpunkten (T1-T4) durchgeführt wird, und
wobei die finale Kenngröße (50) für die unilaterale Anhidrose anhand der Werte (k11-k14, k21-k24) der ersten und der zweiten Kenngröße (23, 24) zu den besagten Zeitpunkten (T1, T2, T3, T4) ermittelt wird.

10. Verfahren nach Anspruch 9 in Verbindung mit Anspruch 8,
wobei für wenigstens einige der besagten Zeitpunkte (T1- T4) ein Korrelationsmaß (MC) der betreffenden Werte (k11-k14, k21-k24) der ersten Kenngröße (23) und der zweiten Kenngröße (24) ermittelt wird, und
wobei die finale Kenngröße (50) für die unilaterale Anhidrose anhand des für die besagten Zeitpunkte (T1-T4) ermittelten Korrelationsmaßes (MC) ermittelt wird.

11. Verfahren nach Anspruch 10,
wobei die finale Kenngröße (50) für die unilaterale Anhidrose als ein Wahrscheinlichkeitswert (P) aus dem für die besagten Zeitpunkte (T1-T4) ermittelten Korrelationsmaß (MC) ermittelt wird, oder
wobei die finale Kenngröße (50) für die unilaterale Anhidrose als ein binärer Wert (B) abhängig von einem Unterschreiten eines unteren Grenzwertes (U) durch das für die besagten Zeitpunkte (T1-T4) ermittelten Korrelationsmaß (MC) ermittelt wird.

12. Verfahren nach einem der vorhergehenden Ansprüche,
wobei eine Benachrichtigung an den Träger und/oder an eine Pflegeperson des Trägers und/oder einen ärztlichen Dienst ausgegeben wird, wenn die finale Kenngröße (50) für die unilaterale Anhidrose einen kritischen Wert einnimmt.

13. Anordnung (1) zum Überprüfen einer menschlichen Körperfunktion, umfassend ein am Kopf (3) tragbares System (2) mit einem ersten Sensor (21) und einem zweiten Sensor (22), und eine Steuereinheit (36),
wobei der erste Sensor (21) im bestimmungsgemäßen Betrieb des Systems (2) auf einer lateralen ersten Seite (9) eines Kopfes (3) eines Trägers angeordnet ist, und der zweite Sensor (22) auf einer der ersten Seite (9) gegenüberliegenden zweiten Seite (10),
wobei der erste Sensor (21) und der zweite Sensor (22) dazu eingerichtet sind, jeweils eine erste Kenngröße (23) bzw. zweite Kenngröße (24) zu messen, welche Aufschluss über einen an besagter ersten Seite (23) bzw. zweiten Seite (24) des Kopfes (3) abgegebenen Schweiß gibt, und
wobei die Steuereinheit (36) dazu eingerichtet ist, anhand der ersten Kenngröße (23) und der zweiten Kenngröße (24) eine finale Kenngröße (50) für eine unilaterale Anhidrose eines Trägers des besagten Systems (2) zu ermitteln.

14. Anordnung (1) nach Anspruch 13,
wobei das System (2) gegeben ist durch ein binaurales Hörsystem (6) mit einem ersten Hörinstrument (7) und einem zweiten Hörinstrument (8), und
wobei der erste Sensor (21) im ersten Hörinstrument (7) und der zweite Sensor (22) im zweiten Hörinstrument (8) angeordnet ist.

15. Anordnung (1) nach Anspruch 13 oder Anspruch 14,
wobei die Steuereinheit (36) in einem externen Gerät (4) angeordnet ist, welches mit dem am Kopf (3) tragbaren System (2) datentechnisch verbindbar ist.

16. Anordnung (1) nach einem der Ansprüche 13 bis 15,
wobei der erste Sensor (21) und/oder der zweite Sensor (22) jeweils gegeben ist durch
- einen photoplethysmographischen Sensor und/oder
- einen kapazitiven Sensor und/oder
- einen Elektromyographie-Sensor und/oder
- einen Elektrokardiographie-Sensor und/oder
- einen Elektroenzephalographie-Sensor und/oder
- einen Feuchtigkeitssensor und/oder
- einen Hautleitfähigkeitssensor und/oder
- einen Reflektometriesensor und/oder
- einen Cerumen-Sensor und/oder
- eine Anordnung, welche eine Lichtquelle und einen Photodetektor aufweist.

17. Verwendung eines am Kopf (3) tragbaren Systems (2) mit einem ersten Sensor (21) und einem zweiten Sensor (22) zur Detektion einer unilateralen Anhidrose eines Trägers des Systems (2),
wobei der erste Sensor (21) im bestimmungsgemäßen Betrieb des Systems (2) auf einer lateralen ersten Seite (9) eines Kopfes (3) des Trägers angeordnet ist, und der zweite Sensor (22) auf einer der ersten Seite (9) gegenüberliegenden zweiten Seite (10),
wobei der erste Sensor (21) und der zweite Sensor (22) dazu eingerichtet sind, jeweils eine erste Kenngröße (23) bzw. zweite Kenngröße (24) zu messen, welche Aufschluss über einen an besagter ersten Seite (9) bzw. zweiten Seite (10) des Kopfes (3) abgegebenen Schweiß gibt, und
wobei anhand der ersten Kenngröße (23) und der zweiten Kenngröße (24) eine finale Kenngröße (50) für eine unilaterale Anhidrose des Trägers ermittelt wird.
